# EUROPEAN PATENT APPLICATION

(11) **EP 2 022 470 A1**
(43) Date of publication of application: **11.02.2009**
(21) Application number: 07015554.4
(22) Date of filing: 07.08.2007
(51) Int. Cl.: A61K 8/34, A61K 8/41, A61K 8/49, A61Q 5/06, A61K 8/44

(54) **Colouring composition**

(71) Applicant: KPSS-Kao Professional Salon Services GmbH, 64297 Darmstadt (DE)
(72) Inventor: Hoffmann, Martin, 64673 Zwingenberg (DE); Tietjen, Ilka, 69207 Sandhausen (DE); Bistram, Vera, 64683 Einhausen (DE); Hopf, Renate, 63303 Dreieich (DE)

(57) **Abstract**

The present invention is related to an aqueous colouring composition for keratin fibres especially human hair. Accordingly, the object of the present invention is an aqueous hair colouring composition comprising at least one cationic direct dye, at least one fatty alcohol, at least one emulsifier and having a pH in the range of 5.2 to 7.5.

## Description

The present invention is related to an aqueous colouring composition for keratin fibres especially human hair.

Hair colouring has been widely practiced for ages. The colorations are divided into two main groups, the first being permanent colouration based on mainly oxidative hair dyes which penetrate into hair and polymerize, and the second is based on direct dyes which is, without excluding any penetration, mainly adsorbed onto hair and widely based on cationic and neutral dyes. In the latter, recently compositions based on anionic direct dyes have also been made available by the applicant which deliver brilliant, shiny and long lasting colours,

It is usually believed that colour intensity and durability with direct dyes is not as satisfying as achieved with oxidative dyes. Furthermore, it is believed that dye adsorption onot hair surface is governed by electrostatic interaction of the cationic dye and anionic hair surface and therefore, almost all conditioning compositions with colour enhancing effect have been formulated in the acidic pH range, i.e. below pH 5.0.

The inventors of the present invention have surprisingly found out that a: composition comprising at least one cationic direct dye, at least one fatty alcohol and at least one emulsifier and having a pH of 5.2 to 7.5 colours hair more intensively than a composition having pH below 5.0. Colours so obtained are excellently shiny and have vibrancy and brilliance. In addition the compositions of the present invention conditions hair in terms of combability, elasticity, smoothness and softness.

Accordingly, the object of the present invention is an aqueous hair colouring composition comprising at least one cationic direct dye, at least one fatty alcohol, at least one emulsifier and having a pH in the range of 5.2 to 7.5.

Another object of the present invention is use of composition comprising at least one direct dye at least one fatty alcohol, at least one emulsifier and having a pH in the range of 5.2 to 7.5 for intensive colouring hair.

Compositions of the present invention comprise at least one cationic direct dye. In principal any cationic direct dye suitable for colouring keratin fibres especially hair is useful within the scope of the present invention. Special mention is made to cationic hair dyes disclose in international patent applications of Ciba-Geigy with the publication numbers WO 95/15144 A1 and WO 95/01772 A1. Their content is included by reference in the present application.

Non-limiting examples are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and their salts such as chloride, methosulfate, bromide etc. and mixtures thereof.

Composition of the present invention may comprise further direct dyes such as anionic dyes, neutral nitro dyes and plant derived dyes in addition to the cationic direct dyes. Care should be taken about the compatibility of the dyes when direct dyes in mixture of several categories are intended to be used.

Suitable non-limiting examples to anionic dyes are Acid Black 1. Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and their mixtures.

Suitable non-limiting examples to nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow Neo.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and their mixtures.

Plant dyestuffs may also be used as hair colorant within the meaning of the present invention for example henna (red or black), alkanna root, laccaic acid, indigo, logwood powder, madder root and rhubarb powder, etc.

Concentration of direct dyes in the compositions of the present invention is within the range of 0.001 to 5%, preferably 0,01 to 4% and more preferably 0.05 to 3%, and most preferably 0.1 to 2% by weight, calculated to total composition.

Compositions of the present invention comprise at least one fatty alcohol with linear of branched alkyl chain. Suitable ones are fatty alcohols having 12 to 22 C atoms in its alkyl chain. Examples are myristyl alcohol, cetyl alcohol, stearyl alcohol, behenyl alcohol and their mixtures. Preferred are cetyl, stearyl and behenyl alcohol and their mixtures i.e. cetearyl alcohol. Fatty alcohols are included into the compositions of the present invention at a concentration of 0.1 to 20%, preferably 0.5 to 15% and more preferably 1 to 10% by weight calculated to total composition.

Compositions of the present invention comprise at least one emulsifier, As a rule any surfactant wit emulsifying power should be useful for the compositions of the present invention. Preferred are cationic and non-ionic surfactants and their mixtures. From the cationic surfactants those with mono long alkyl chains are especially suited as emulsifier. Anionic and amphoteric surfactants are not preferred surfactants as emulsifiers for the compositions of the present invention.

As a rule any cationic surfactant is suitable for the compositions of the present invention. With the term cationic surfactant it is meant that the surfactant carries a cationic charge when used in the compositions. In other words, compounds having no cationic charge but when added into the compositions protonate and therewith become cationic are also included within the definition of cationic surfactant. An example to such may be stearyldimethylamine and PEG-2 Cocamine are as a compound not carrying a cationic charge but when used in a composition having acidic pH becomes cationic by protonation.

Preferably at least one cationic surfactant is selected from the compound with the general formula 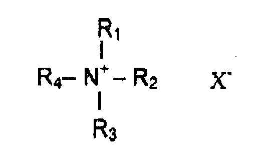 where R₁s a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Suitable cationic surfactants are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, stearamidopropyldimethylamoonium chloride, dipalmitoyl dimonium chloride, distearyl dimethyl ammonium chloride, stearamidopropyl trimonuim chloride.

From the above mentioned cationic surfactants mono long chain alkyl ones are emulsifiers and conditioners and the di long chain alkyl ones are conditioners and are not preferred emulsifiers.

Further examples to the cationic surfactants are so called esterquats available on the market, for example, under the trade names "Schercoquat^{®}, "Dehyquart^{®}L80" and "Tetranyl^{®}". Still further examples are so called amidoquats again available on the market, for example, under the trade name "INCROQUAT^{à} HO" or "OCS".

Suitable non-ionic surfactants are alkyl polyglucosides of the general formula

R₇-O-(R₈O)ₙO-Zx

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono-and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 50, preferably about 10 and about 30.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

In a preferred embodiment of the present invention, colouring compositions comprise at least one cationic surfactant with mono long chain alkyl and at least one non-ionic surfactant as emulsifiers at any ratio. Preferred non-ionic emulsifier is fatty alcohol ethoxylates as mentioned above

Total surfactant concentration varies between 0.1 and 15%, preferably 0.5 and 10%, and more preferably 1 to 7.5% by weight calculated to total composition.

Compositions of the present invention have a pH in the range of 5.2 to 7.5, preferably 5.5 to 7.0, more preferably 6.0 to 7.0.

In a preferred embodiment of the present invention, colouring composition comprise at least one fatty acid alky ester at a concentration of 0.1 to 5%, preferably 0.2 to 3% by weight calculated to total composition. The fatty acid alkyl esters preferred are isopropyl myristate, isopropyl palmitate, isopropyl stearate and i isopropyl sostearate, oleyl oleate, isocetyl stearate, hexyl laurate, dibutyl adipate, dioctyl adipate, myristyl myristate, oleyl erucate, cetyl palmitate. Most preferred are isopropyl myristate, isopropyl palmitate, isopropyl stearate and isopropyl isostearate and their mixtures.

Compositions of the present invention can comprise additionally hair conditioning compounds such as oils, cationic polymers, non-ionic substances. Oils as conditioners according to the present invention are selected from silicone oils either volatile or non-volatile, natural and synthetic oils. Among silicone oils those can be added to the compositions include either volatile or non-volatile dimethicone, dimethiconol, polydimethylsiloxane, DC fluid ranges from Dow Corning, cyclosiloxanes such as DC 245, arylated silicones such as phenyltrimethicone available from Dow Corning under trade name DC 556 and phenyl methicone, , diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane.

Synthetic oils include mineral oil such as paraffin oil and petrolatum.

Natural oils suitable are such as olive oil, almond oil, avocado oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₉ CO (OCH₂CH₂)ₙ OH

R₉ CO (OCH₂CH₂)ₙ O OC R₁₀

where R₉ and R₁₀ are independent from each other saturated, unsaturated or branched or non-branched alkyl chain with 7 to 21 C atoms and n is typically 2 - 100.

Composition of the present invention can comprises cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46, Polyquaternium 67, Polyquaternium 72, Polyquaternium-24.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quatemium-27, Quaternium-30, Quaternium-33, Quaterntium-53, Quaternium-60, Quaternium-61, Quaternium-72, Quaternium-78, Quaternium-80, Quaternium-81, Quaternium-82, Quaternium-83 and Quatemium-84.

In this context, reference is also made to the cationic polymers disclose in DE 25 21 960, 28 11 010, 30 44 738 and 32 17 059, as well as to the products described in EP-A 337 354 on pages 3 to 7. It is also possible to use mixtures of various cationic polymers.

Further cationic polymers are so called aminated silicones such as amodimethicone. The cationic polymers also include the quatemized products of graft polymers from organopolysiloxanes and polyethyl oxazolines described in EP-A 524 612 and EP-A 640 643.

Concentration range for any of the additional conditioners mentioned above is in the range of 0.01 to 10% by weight, preferably 0.05 - 7.5% by weight, more preferably 0.1 - 5% by weight calculated to the total composition.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R}". Extracts and the preparation thereof are also described in "Hagers Handbuch der pharmazeutischen Praxis", 4th Ed.

The compositions can contain one or more organic solvents such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvent can be in the range of 1 to 40%, preferably 1 to 25% by weight, calculated to total composition.

Compositions of the present invention can comprise UV filters for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-solfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition.

The compositions of the present invention can comprise one or more hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide,

Preferred fatty acids are those with 10 to 24 carbon atoms and especially with 16 to 24 carbon atoms.

Sterols,especially the phytosterols, are as well preferred hair restructuring agents as disclosed in the above mentioned german patent Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition, Phytosterol concentration of the conditioners is less than 1% and preferably in the range of 0.01 to 0.5% by weight calculated to the total weight of the composition. It should be noted without limiting the use of those ingredients the effect of those hair restructuring ingredients is especially elevated when used in combination with penetration enhancers.

Accordingly the first object of the present invention is a conditioning composition for hair characterised in that it comprises at least one optical brightener and at least one ubichinone of the following formula 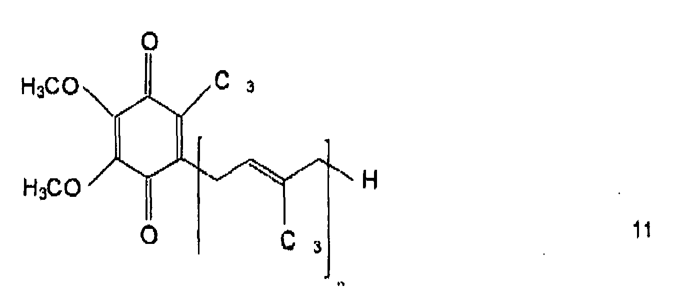 where n is a number between 1 and 10.

Furthermore, compositions of the present invention can comprise particulate matter dispersed in it such as synthetic mica. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and are known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.25 to 2.5% by weight calculated to total composition.

Compositions of the present invention may also include thickening agents such as polymers. The thickening agents include any polymer either natural or synthetic thickening aqueous composition. Examples are cellulose and its derivatives such as hydroxyethylcellulose, guar and its derivatives such as hydroxypropyl guar. In the selection of the thickening agent compatibility with any other components of the formulation should carefully be examined.

The gelling agents include polymers either synthetic or natural forming shear thinning compositions. Examples to the natural polymers are xanthan gum and its derivatives. Synthetic shear thinning polymers may be those of acrylate polymers commercially available for example under trade name Carbopol. In the selection of the geling agent compatibility with any other components of the formulation should carefully be examined.

It should be noted that gelling and thickening agents can also be used in mixture. Concentration of the thickening and/or gelling agents should be in the range of 0.05 to 5%, preferably 0.1 to 2.5% by weight calculated to total content.
Following examples are to illustrate the invention but not to limit.

### Example 1

| | % by weight |
|---|---|
| Cetearyl alcohol | 4.0 |
| Behentrimonium chloride | 0.8 |
| Steartrimonium chloride | 0.6 |
| Isopropyl palmitate | 0.5 |
| Basic red 76 | 0.15 |
| Basic brown 16 | 0.20 |
| Citric acid/Sodium hydroxide | q.s. to pH 6.8 |
| Fragrance, preservative | q.s. |
| Water | to 100 |

The above composition was prepared by emulsifying fatty alcohol and the two cationic surfactants together with isopropyl palmitate in water at 70°C and after cooling down to approximately 40°C, dye solution in water and remaining components were added. Finally pH was adjusted.

For comparative purposes the above composition was produced in the same way but the pH was adjusted to 3.8.

Two hair tresses of same colour, medium blond were coloured at room temperature with the above composition, inventive and comparative, and after drying L, a and b values were measured with a laboratory equipment purchased from Minolta. From these values DE value was calculated to show the colour difference

The L, a and b values of the tress coloured with the inventive composition are 21.86, 17.68 and 11.38, respectively. The L, a and b values of the tress coloured with the comparative composition are 28.19, 22.90 and 20.35 respectively.. From the set of the values a DE value of 12.16 is calculated which show a significant colour difference.

From the above it is clear that the inventive composition produces intensive colour.

At the same time with a half side test it was proven that the inventive composition has a higher conditioning level than the comparative one. It was proven that combing of hair treated with inventive composition was much easy.

Similar results were observed with the examples below.

### Example 2

| | % by weight |
|---|---|
| Cetearyl alcohol | 8.0 |
| Behentrimonium chloride | 2.0 |
| Ceteareth-20 | 1.0 |
| Basic red 51 | 0.2 |
| Citric acid/Sodium hydroxide | q.s. to pH 6.6 |
| Fragrance, preservative | q.s. |
| Water | to 100 |

### Example 3

| | % by weight |
|---|---|
| Cetearyl alcohol | 10.0 |
| Behentrimonium chloride | 2.0 |
| Ceteareth-20 | 1.0 |
| Isopropyl palmitate | 0.8 |
| Basic orange 31 | 0.05 |
| Basic yellow 87 | 0.10 |
| Citric acid/ammonium hydroxide | q.s. to pH 6.5 |
| Fragrance, preservative | q.s. |
| Water | to 100 |

### Example 4

| | % by weight |
|---|---|
| Cetearyl alcohol | 8.0 |
| Ceteareth-20 | 3.0 |
| Isopropyl stearate | 0.4 |
| Propylene glycol | 2.0 |
| Basic red 51 | 0.5 |
| Ammonium hydroxide/citric acid | q.s. to pH 6,8 |
| Fragrance, preservative | q.s. |
| Water | to 100 |

Similar results were observed with the above composition as in Example 3.

### Example 5

| | % by weight |
|---|---|
| Cetearyl alcohol | 10.0 |
| Cetrimonium chloride | 3.0 |
| Isopropyl palmitate | 0.5 |
| Propylene glycol | 2.0 |
| Phenyl trimethicone | 0.5 |
| Dimethicone | 0.5 |
| Basic red 51 | 0.5 |
| Acid red 52 | 0.5 |
| Ammonium hydroxide/citric acid | q.s. to pH 6.7 |
| Fragrance, preservative | q.s. |
| Water | to 100 |

### Example 6

| | % by weight |
|---|---|
| Cetearyl alcohol | 8.0 |
| Steartrimonium chloride | 2.0 |
| Stearamidopropyl trimonium chlorde | 1.5 |
| Propylene glycol | 2.0 |
| Dimethicone | 0.5 |
| Basic red 51 | 0.1 |
| HC Red 3 | 0. |
| Ammonium hydroxide | q.s. to pH 6.8 |
| Fragrance, preservative | q.s. |
| Water | to 100 |

### Example 7

In this example, hair was bleached first with a bleaching composition according to EP 560 088 B1 according to the method as disclosed in the description of the patent and bleaching composition was rinsed off from hair. In the second step composition according to Example 4 as given above was applied onto hair. Hair streaks obtained showed excellent shiny, brilliant and vibrant intensive red colour.

### Example 8

| | % by weight |
|---|---|
| Cetearyl alcohol | 10.0 |
| Behentrimonium chloride | 2.0 |
| Ceteareth-20 | 0.9 |
| Basic red 76 | 0.1 |
| Basic red 51 | 0.1 |
| Ethyl hexyl methoxy cinnamate | 0.3 |
| Citric acid/Sodium hydroxide | q.s. to pH 6.5 |
| Fragrance, preservative | q.s. |
| Water | to 100 |

### Example 9

| | % by weight |
|---|---|
| Cetearyl alcohol | 10.0 |
| Cocamide MEA | 4.0 |
| Sodium lauryl sulphate | 1.5 |
| Propylene glycol | 2.0 |
| Trimethyl pentaphenyl trisiloxane | 1.0 |
| Vitis vinifera* | 0.1 |
| Basic red 51 | 0.5 |
| Acid red 52 | 0.5 |
| Lactic acid | q.s. to pH 3.5 |
| Fragrance, preservative | q.s. |
| Water | to 100 |

| | |
|---|---|
| *: dry matter | |

### Example 10

| | % by weight |
|---|---|
| Cetearyl alcohol | 10.0 |
| Cetrimonium chloride | 1.0 |
| Polyquaternium-10 | 0.5 |
| Hydroxyethylcellulose | 0.5 |
| Ubichinone | 0.1 |
| Basic red 51 | 0.2 |
| Lactic acid/sodium hydroxide | q.s. to pH 6.7 |
| Fragrance, preservative | q.s. |
| Water | to 100 |

### Example 11

| | % by weight |
|---|---|
| Cetearyl alcohol | 10.0 |
| Cetrimonium chloride | 1.0 |
| Ceteareth-30 | 2.0 |
| Polyquaternium-10 | 0.5 |
| Ubichinone | 0.1 |
| Isopropyl myristate | 0.5 |
| Basic red 51 | 0.1 |
| Benzophenone-3 | 0.2 |
| Lactic acid/sodium hydroxide | q.s. to pH 6.8 |
| Fragrance, preservative | q.s. |
| Water | to 100 |

### Example 12

| | % by weight |
|---|---|
| Cetearyl alcohol | 10.0 |
| Ceteareth-20 | 3.0 |
| Cetrimonium chloride | 0.5 |
| Polyquaternium-10 | 0.75 |
| Dimethicone | 1.0 |
| Isopropyl palmitate | 0.5 |
| Basic red 51 | 0.2 |
| Benzophenone-3 | 0.2 |
| Lactic acid/sodium hydroxide | q.s. to pH 6.6 |
| Fragrance, preservative | q.s. |
| Water | to 100 |

### Example 13

| | % by weight |
|---|---|
| Cetearyl alcohol | 10.0 |
| Ceteareth-20 | 3.0 |
| Cetrimonium chloride | 0.5 |
| Polypuaternium-10 | 0.5 |
| Amodimethicone | 0.5 |
| Isopropyl palmitate | 0.2 |
| Synthetic fluorphologopite* | 1.0 |
| Basic red 51 | 0.2 |
| Benzophenone-3 | 0.2 |
| Lactic acid/sodium hydroxide | q.s. to pH 6.5 |
| Fragrance, preservative | q.s. |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

### Example 14

| | % by weight |
|---|---|
| Cetearyl alcohol | 10.0 |
| Ceteareth-20 | 3.0 |
| Cetrimonium chloride | 0.5 |
| Polyquaternium-10 | 0.5 |
| Polysilicone-9 | 0.5 |
| Isopropyl stearate | 0.5 |
| Basic red 51 | 0.15 |
| Basic orange 31 | 0.1 |
| Benzophenone-3 | 0.2 |
| Lactic acid/sodium hydroxide | q.s. to pH 4.0 |
| Fragrance, preservative | q.s. |
| Water | to 100 |

## Claims

1. Aqueous composition for colouring hair **characterised in that** it comprises at least one cationic direct dye, at least one fatty alcohol, at least one emulsifier and having a pH in the range of 5.2 to 7.5.

2. Composition according to claim 1 **characterised in that** it comprises as an emulsifier a cationic surfactant or a non-ionic surfactant or mixtures thereof.

3. Compositions according to claims 1 and 2 cationic surfactant is selected from the compounds with mono long alkyl chain according to the formula 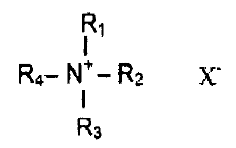 where R₁s a saturated or unsaturated, branched or non-branched alkyl chain with 8-22 C atoms or
R₅CO NH (CH₂)ₙ
where R₅ is saturated or unsaturated, branched or non-branched alkyl chain with 7-21 C atoms and n has typical value of 0 - 4 or
R₆CO O (CH₂)ₙ
where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or
R₅CO NH (CH₂)ₙ
where R₅ is saturated or unsaturated, branched or non-branched alkyl chain with 7-21 C atoms and n has typical value of 0 - 4 or
R₆ CO O (CH₂)ₙ
where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

4. Compositions according to claim 3 **characterised in that** cationic surfactants are selected from cetyl trimethyl ammonium chloride, myristoyl trimethyl ammonium chloride, behentrimonium chloride, trimethyl cetyl ammonium bromide, stearyl trimethyl ammonium chloride, stearamidopropyl dimethyl ammonium chloride, stearamidopropyl trimonuim chloride.

5. Composition according to any of the preceding claims **characterised in that** nonionic surfactant is a fatty alcohol ethoxylate.

6. Composition according to any of the preceding claims **characterised in that** it comprises at least one fatty acid alkyl ester at a concentration of 0.1 to 5% by weight calculated to total composition.

7. Composition according to claim 6 **characterised in that** it comprises isopropyl myristate, isopropyl palmitate, isopropyl stearate and isopropyl isostearate and their mixtures.

8. Composition according to any of the preceeding claims **characterised in that** it has a pH between 6.0 and 7.0.

9. Composition according to any of the preceding claims **characterised in that** it comprises additional hair conditioning compounds selected from oils, cationic polymers, non-ionic substances.

10. Composition according claim 7 **characterised in that** it comprises silicone oil as an oil.

11. Composition according to any of the preceding claims **characterised in that** comprises one or more organic solvent(s).

12. Composition according to any of the preceding claims **characterised in that** comprises at least one UV filter.

13. Composition according to any of the preceding claims **characterised in that** it comprises it comprises at least one ubichinone of the following formula 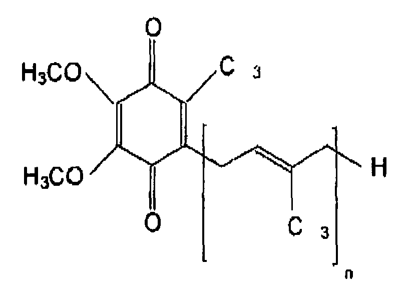 where n is a number between 1 and 10.

14. Use of a composition according to claims 1 to 11 for colouring hair.
